## Europäisches Patentamt

## European Patent Office

## Office européen des brevets

(19)

(11) Publication number: **0 244 491
B1**

(12)

# EUROPEAN PATENT SPECIFICATION

(45) Date of publication of the patent specification:
**05.09.90**

(51) Int. Cl.⁵: **A61B 17/32**

(21) Application number: **86105889.9**

(22) Date of filing: **29.04.86**

---

(54) Medical instrument for removing bone.

---

(43) Date of publication of application:
**11.11.87 Bulletin 87/46**

(45) Publication of the grant of the patent:
**05.09.90 Bulletin 90/36**

(84) Designated Contracting States:
**DE FR GB**

(56) References cited:
**US-A- 4 574 803**

(73) Proprietor: **Waldemar Link GmbH & Co,
Barkhausenweg 10, D-2000 Hamburg 63(DE)**

(72) Inventor: **Farley, Daniel K., 148 S. North West Highway,
Barrington Illinois 60010(US)**

(74) Representative: **Glawe, Delfs, Moll & Partner
Patentanwälte, Postfach 26 01 62 Liebherrstrasse 20,
D-8000 München 26(DE)**

---

ACTORUM AG

## Description

This invention relates generally to forceps of the rongeur or bone cutting type. It has long been an object in the art to provide simple, inexpensive forceps capable of easy disassembly and cleaning, and most importantly capable of safe and efficient operation. Prior patents issued in this art have proposed possible solutions. Typical of the prior art is the patent issued to De Vilbiss, U. S. Patent No. 1,040,523, which provides a lever mechanism comprising a handle having two sides arranged to pivot towards one another about a central axis. On extensions of these handle members there is provided a jaw mechanism comprising a first movable jaw arranged to travel within a slot defined within a second movable jaw. Bone seized between the jaws is pulled within the slot as it is cut. A problem associated with this and other mechanisms in the art is that uncontrolled force is applied to the bone surface surrounding the cutting area, such that possible bone breakage and splintering may occur. If operating near the spinal cord, possible nerve damage may result. Further, bone chips cut from the bone structure are not well contained by the instrument and contamination of the wound may result. Finally, with the pivot placed between the jaw mechanism and the lever handles limited mechanical advantage is available, making the instrument tiring to use.

The US-A 4 574 803 discloses a medical cutter particularly adapted for cutting and removing soft tissue, particularly usefull in laparoscopy. Though there is passing reference to cutting bone, the primary purpose of the know device is to cut soft tissue. It comprises a rigid barrel member having a cutting edge defined at one end thereof; a shaft member positioned coaxially within said barrel member and arranged for reciprocal motion therein, said shaft member having a plate member mounted at one extremity thereof transverse to said shaft member and defining, together with a depression in the shaft, a cavity proximate to said plate member; a second cutting edge being formed on said plate member; and lever means for reciprocally translating said shaft member within said barrel to cause said plate to reciprocate with respect to said barrel whereby tissue is trapped between said cutting edges, and to cause said cavity to reciprocate with respect to said barrel whereby severed tissue is captured within said cavity.

It is in object of the present invention to provide an improved instrument providing sufficient force for evenly cutting bone during surgical procedures.

According to the invention, this is achieved by the features of claim 1.

In removing bone, this allows for a cutting action rather than a crushing action, thereby relieving pressure on the plate member and thus reducing the possibility of bone breakage and splintering beyond the cutting area and of breaking said member while cutting bone structures.

Other objects and advantages of the invention will become apparent upon reading the following detailed description and upon reference to the drawings, in which:

Figure 1 is a perspective view partially cut away of the preferred embodiment of the present invention.

Figure 2 is an enlarged perspective view of the distal end of the instrument in operation.

Figure 3 is a side view of the preferred embodiment of the present invention.

Figure 4 is a side view of a shaft member used in the present invention.

Figure 5 is a perspective view of the end of the shaft of Figure 4 viewed along the line AA.

Figure 6 is a perspective view partially cut away of the device of Figure 3 viewed along the line BB.

Fig. 7 is a side view of an alternative embodiment.

Fig. 8 is a sectional view according to line IIX-IIX of Fig. 7 and

Fig. 9 is a sectional view according to line IX-IX of Fig. 7.

## DESCRIPTION OF THE PREFERRED EMBODIMENT

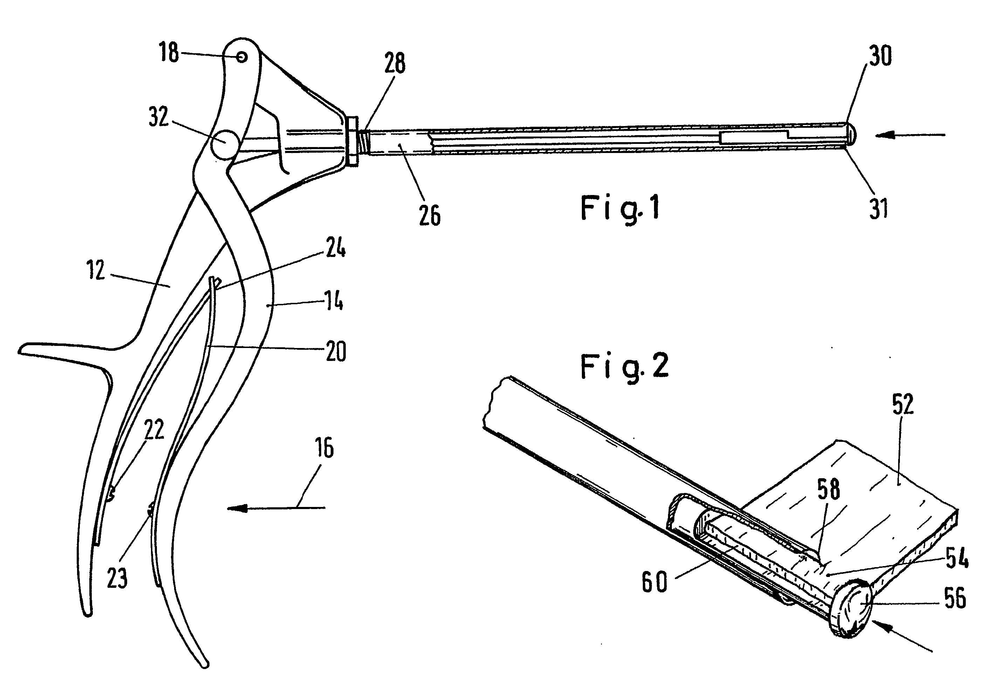

Turning first to Figure 1 there is shown the forceps of the preferred embodiment of the present invention. Specifically there is shown a paired handle member 12 and 14 arranged to be held within the human hand and pulled together in the direction of the arrow 16 much in the same manner as a pair of pliers. This handle pair is arranged to pivot about the axis 18 and each is arranged as shown in the drawing to support and actuate the barrel and shaft cutting mechanism as more fully described below.

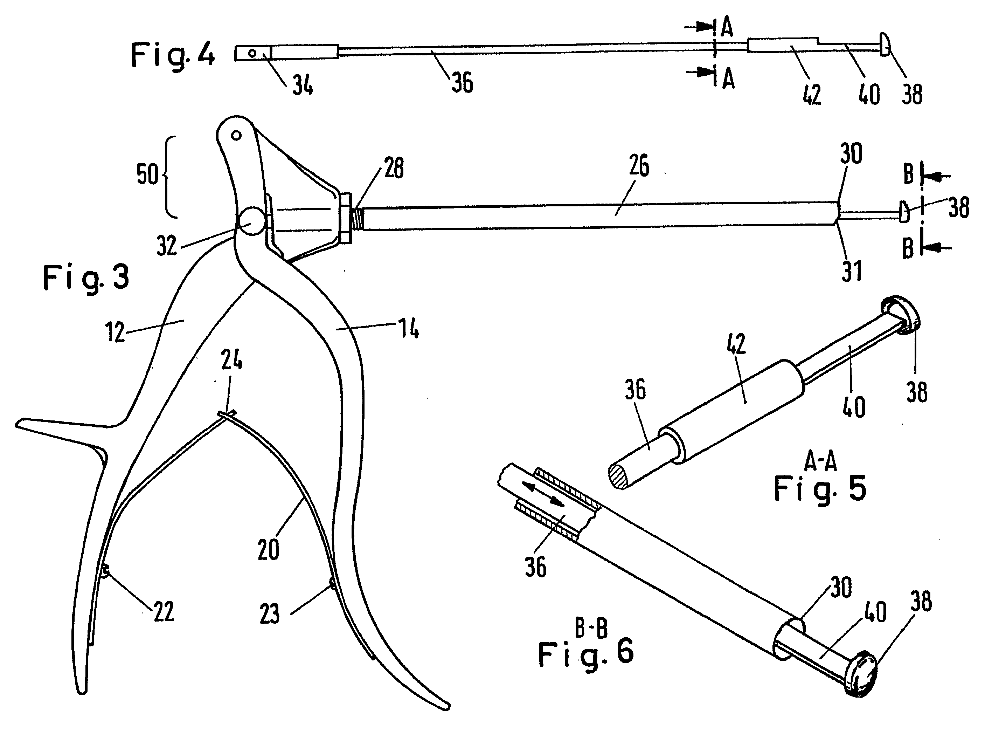

A squeezing force on the paired handle members closes the handles in the direction of the arrow 16, and concurrently compresses the intermediate spring 20 located between the handle pair. This generates a returning force to return the handles to their starting position as shown in Figure 3. The return spring is typically of spring steel made of distinct and separate members, each rigidly attached to respective individual handle members at the lower extremity 22 and 23 respectively, while being hingedly interlocked at the intermediate contacting point 24.

Rigidly attached by screw thread means to the first member of the handle pair there is provided a barrel member 26 comprised of a cylinderical tube having external threads 28 at its point of attachment to the handle, and having at its other extremity a honed edge 30 (more clearly shown in Figure 6) for cutting bone pressed thereagainst. Generally, only the upper edge is honed for cutting with the lower edge 31 being angled away from the end to avoid contact with the reciprocating mechanisms.

Mounted for reciprocal motion within said barrel there is provided a shaft member pivotally attached at axis 32 to said second handle member and arranged to selectively protrude from said barrel at its other extremity. This shaft member (Figure 4) is comprised of a first attachment portion 34 for connection to the pivot mount at the handle, and a force transmission portion 36 for transmitting the force of the handle leverage mechanism remotely to the site of the bone cutting at the distal end of the barrel. Finally, there is a bone cutting and trapping portion comprised of a contoured plate 38 mounted transversely to the distal end of the shaft. This plate is shown with increased thickness in the vicinity of its attachment to the shaft to resist breakage. Within said shaft there is provided a cavity portion 40 (see Figure 5) defined by a depression on such shaft. In the preferred embodiment this cavity is formed by a flat depression represented by a cut away portion of said shaft. Finally, a sealing portion 42 is provided to close the gap between the barrel and the shaft as the shaft end is drawn into the barrel. This sealing portion acts to maintain the barrel free of debris and serves to eject bone fragment following a cutting sequence.

To improve the mechanical advantage of the forceps, increased leverage is provided by attaching the barrel and shaft between the handle pivot and the handle grasping portion where hand pressure is applied during the squeeze. By varying the distance 50 between the barrel and shaft attachment points and the pivot, mechanical advantage can be set to provide comfortable operation during strenuous bone cutting procedures. Additionally, the tendency of the forceps to move during actuation is reduced as the spacing 50 is increased while fine control is retained by keeping the grasped portion of the handle near the barrel and shaft attachment.

In operation (see Figure 2) there is shown a bone segment 52 with an unsevered portion 54 trapped between the shaft and plate 56 and the honed edge of the barrel 58. As the shaft is drawn towards the barrel, severed bone 60 is captured within the barrel. Once the stroke has been completed the forceps are removed from the cutting site, inverted, and the shaft extended from the barrel to facilitate deposit of the severed bone into the proper receptacle.

There has been shown and described an improved forcep or rongeur having a jaw mechanism comprised of a barrel member having a honed cutting edge on one extremity thereof and having a shaft member located within and arranged for reciprocal motion. On the shaft member there is provided a plate member attached at one extremity to trap bone against the cutting edge. A lever mechanism is provided having paired handle levers joined by a pivot and having the barrel and shaft members attached to the handle levers between the handle portion and the pivot. On the shaft member there is further provided a cavity arranged to slide within the barrel progressively as the cutting operation proceeds, gradually drawing severed bone within the capturing cavity.

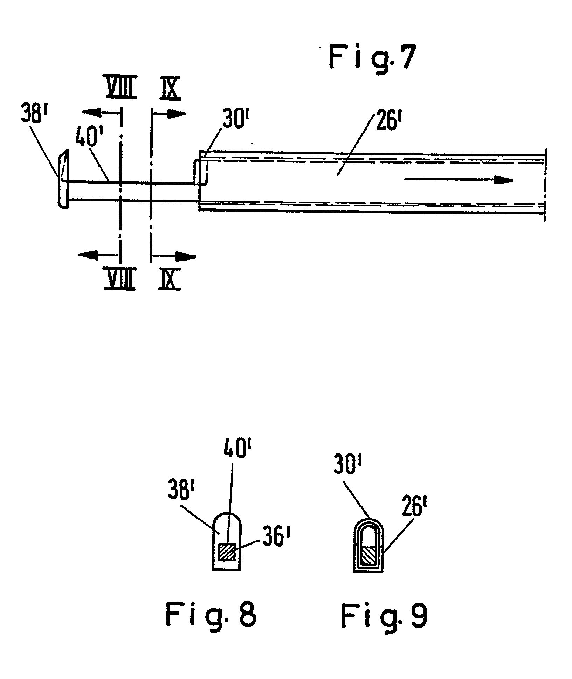

Whereas in the embodiment according to Figs. 1 - 6 the barrel member and the correspondingly contoured plate 38 have a circular cross-sectional shape, in the embodiment of Fig. 7 - 9 this shape is elongate. The cutting edge 30' of the barrel member 26' and the corresponding contour of the plate 38' are defined by two parallel sections and an arcuate section bridging the parallel section. In the region of the cavity portion 40' the shaft 36' has a rectangular cross section. In comparison with the embodiment described with reference to Figs. 1 - 6, this alternative embodiment allows a greater volume of bone to be trapped and severed. Also other cross sectional shapes may be used within the inventive idea.

## Claims

1. A medical instrument for cutting and removing bone comprising: a rigid barrel member (26) having a cutting edge (30, 58) defined at one extremity thereof; a shaft member (36) positioned coaxially within said barrel member and arranged for reciprocal motion therein, said shaft member having a plate member (38, 56) mounted at one extremity thereof transverse to said shaft member and defining, together with a depression in the shaft, a cavity (40) proximate to said plate member; and lever means (12, 14) for reciprocally translating said shaft member within said barrel (a) to cause said plate (38, 56) to reciprocate with respect to said barrel (26) trapping said bone between said cutting edge (30, 58) and said plate (38, 56) and (b) to cause said cavity (40) to reciprocate with respect to said barrel capturing bone severed by said cutting edge within said cavity; said reciprocal translation of said shaft member within said barrel defining an open position and a closed position of said medical instrument; said open position being defined by said plate being spaced from said cutting edge; said barrel remaining stationary while said medical instrument is moved between said open position and said closed position, characterised by that said closed position is defined by said plate transversely abutting said cutting edge such that as said medical instrument is moved from said open position to said closed position, said plate (38, 56) is brought into abutting contact with said cutting edge (30, 58).

2. The medical instrument of claim 1 wherein said shaft member has further defined thereon a sealing portion (42) having a diameter sufficient to provide an effective sealing fit within said barrel for facilitating the capture of severed bone within said barrel and the selective ejectment thereof.

3. The medical instrument of claim 2 wherein said cutting edge is comprised of a honed edge (58) of said barrel (26).

4. The medical instrument of claims 1, 2 or 3 wherein said lever means is comprised of a pivoted handle mechanism having pivoted paired handle portions (12, 14) wherein said shaft and said barrel are connected to said handle mechanism at a point (32) between said paired handle portions and said pivot (18).

## Patentansprüche

1. Medizinisches Instrument zum Schneiden und Entfernen von Knochen, welches umfaßt: ein starres Rohrglied (26), an dessen einem Ende eine Schneidkante (30, 58) gebildet ist; ein Stangenglied (36), das koaxial in dem Rohrglied hin und zurückbeweglich angeordnet ist und an seinem einen Ende ein Plattenglied (38, 56) trägt, das quer zu dem Stangenglied angebracht ist und gemeinsam mit einer dem Plattenglied nahegelegenen Vertiefung (40) in der Stange eine Höhlung (40) bildet; und Hebelmittel (12, 14) zum Hin- und Zurückbewegen des Stangenglieds innerhalb des Rohrs, um (a) die genannte Platte (38, 56) im Verhältnis zu dem Rohr (26) vor- und zurückzubewegen, wobei der genannte Knochen zwischen der Schneidkante (30, 58) und der genannten Platte (38, 56) gefangen wird, und um (b) die genannte Höhlung (40) im Verhältnis zu dem Rohr vor- und zurückzubewegen, wobei von der Schneidkante abgetrenntes Knochenmaterial innerhalb der Höhlung eingefangen wird; wobei die Vor- und Zurückbewegung des Stangenglieds innerhalb des Rohrs eine offene und eine geschlossene Stellung des medizinischen Instruments bildet; wobei in der offenen Stellung die genannte Platte Abstand von der genannten Schneidkante aufweist; wobei das genannte Rohr stationär bleibt, während das medizinische Instrument zwischen der genannten offenen und geschlossenen Position bewegt wird, dadurch gekennzeichnet, daß in der geschlossenen Stellung die genannte Platte quer an der genannten Schneidkante in solcher Weise anliegt, daß bei der Bewegung des medizinischen Instruments aus der offenen Stellung in die geschlossene Stellung die genannte Platte (38, 56) in Anschlagkontakt an der genannten Schneidkante (30, 58) gebracht wird.

2. Medizinisches Instrument nach Anspruch 1, dadurch gekennzeichnet, daß an dem genannten Stangenglied ferner ein Dichtteil (42) gebildet ist, der einen zur Bildung einer wirksamen Dichtpassung innerhalb des Rohrs ausreichenden Durchmesser aufweist, um das Erfassen von abgetrenntem Knochengewebe innerhalb des Rohrs und dessen gesondertes Auswerfen zu erleichtern.

3. Medizinisches Instrument nach Anspruch 2, dadurch gekennzeichnet, daß die genannte Schneidkante von einer geschliffenen Kante (58) des Rohrs (26) gebildet ist.

4. Medizinisches Instrument nach Anspruch 1, 2 oder 3, dadurch gekennzeichnet, daß die genannten Hebelmittel einen schwenkbaren Griffmechanismus umfassen, der ein Paar von gegeneinander schwenkbaren Griffteilen (12, 14) aufweist, wobei die genannte Stange und das genannte Rohr mit dem Griffmechanismus an einem Punkt (32) zwischen dem Paar von Griffteilen und dem genannten Schwenkpunkt (18) verbunden sind.

## Revendications

1. Instrument médical pour couper et enlever de l'os, comprenant: un tube rigide (26) ayant un bord de coupe (30, 58) à une de ses extrémités; un bras (36) placé coaxialement dans ledit tube et prévu pour s'y déplacer selon un mouvement de va-et-vient, ledit bras comportant une plaque (38, 56) montée à une de ses extrémités transversalement audit bras et délimitant, avec une diminution de taille du bras, une cavité (40) proche de ladite plaque, et un levier (12, 14) pour déplacer selon un mouvement de va-et-vient ledit bras dans ledit tube a) pour que ladite plaque (38, 56) se déplace selon un mouvement de va-et-vient par rapport audit tube (26) en coiçant l'os entre ledit bord de coupe (30, 58) et ladite plaque (38, 56) et b) pour que ladite cavité (40) se déplace selon un mouvement de va-et-vient par rapport audit tube en emprisonnant dans ladite cavité l'os détaché par ledit bord de coupe; ledit mouvement de va-et-vient du bras dans le tube définissant une position ouverte et une position fermée dudit instrument médical; ladite position ouverte correspondant au fait que ladite plaque est espacée dudit bord de coupe; ledit tube restant stationnaire lorsque ledit instrument médical est déplacé entre ladite position ouverte et ladite position fermée, caractérisé en ce que ladite position fermée correspond au fait que ladite plaque est en butée transversale contre ledit bord de coupe de telle sorte que, quand ledit instrument médical est amené de la position ouverte à la position fermée, ladite plaque (38, 56) soit amenée en contact de butée avec ledit bord de coupe (30, 58).

2. Instrument médical selon la revendication 1, dans lequel ledit bras comporte également un tronçon d'obturation (42) ayant un diamètre suffisant pour fournir un ajustement d'obturation efficace dans ledit tube pour faciliter l'emprisonnement de l'os découpé dans ledit tube et son éjection sélective.

3. Instrument médical selon la revendication 2, dans lequel ledit bord de coupe est un bord affilé (58) dudit tube (26).

4. Instrument médical selon les revendications 1, 2 ou 3, dans lequel ledit levier est formé par un mécanisme de poignée à pivot ayant des éléments de poignée (12, 14) appariés à pivot dans lequel ledit bras et ledit tube sont reliés audit mécanisme de poignée en un point (32) situé entre lesdits éléments de poignée appariés et ledit pivot (18).

18

32

12

22

23

28

26

30

31

16

24

14

20

**Fig.1**

**Fig.2**

52

58

54

56

60

Fig. 3

Fig. 4

A-A
Fig. 5

B-B
Fig. 6

Fig.7

Fig. 8　　Fig. 9